(19) Europäisches Patentamt
European Patent Office
Office européen des brevets

(11) Publication number: **0 112 067**
**A2**

(12) # EUROPEAN PATENT APPLICATION

(21) Application number: 83307071.7

(22) Date of filing: 18.11.83

(51) Int. Cl.³: C 07 D 405/04
C 07 D 307/90, A 61 K 31/505

(30) Priority: 18.11.82 JP 203482/82

(43) Date of publication of application: 27.06.84 Bulletin 84/26

(84) Designated Contracting States: BE CH DE FR IT LI NL SE

(71) Applicant: SHIONOGI & CO., LTD.
12, Dosho-machi 3-chome Higashi-ku
Osaka 541(JP)

(72) Inventor: Kamata, Susumu
1-18-14, Hikarigaoka
Takarazuka-shi Hyogo(JP)

(72) Inventor: Haga, Nobohiro
3-8-23-616, Nanko-Naka Suminoe-ku
Osaka-shi Osaka(JP)

(72) Inventor: Nagata, Wataru
6-10, Kawahigashi-cho
Nishinomiya-shi Hyogo(JP)

(72) Inventor: Matsui, Takeaki
123-B-305, Saidera
Suita-shi Osaka(JP)

(74) Representative: Bizley, Richard Edward et al,
BOULT, WADE & TENNANT 27 Furnival Street
London EC4A 1PQ(GB)

(54) **Phthalidylammonium compounds and their production, and their use in the production of 1-phthalidyl-5-fluorouracil derivatives and formulations containing such derivatives.**

(57) Novel phthalidylammonium compounds (IV) are intermediates in a highly selective and high yield process for producing 1-phthalidyl-5-fluorouracil derivatives of formula (I) which are antitumour agents. The process comprises reacting a phthalidyl compound (II) with an amine (III) to yield the quaternary ammonium salts (IV), and then reacting the latter with 5-fluorouracil as follows:

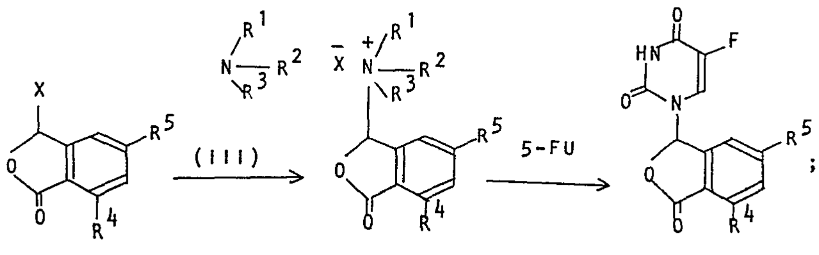


(II) (IV) (I)

in which X is a leaving group; $R^1$, $R^2$ and $R^3$ each independently represents alkyl or $R^1$ represents alkyl and $R^2$ and $R^3$ taken together with the adjacent nitrogen atom form a saturated 5- or 6-membered ring which may contain oxygen or

./...

EP 0 112 067 A2

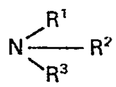

represents quinuclidine; and $R^4$ and $R^5$ each independently represents hydrogen, trialkylsilyloxy, alkoxy, nitro, cyano, carboxy or alkoxycarbonyl.

PHTHALIDYLAMMONIUM COMPOUNDS AND THEIR PREPARATION, AND THEIR USE IN THE PRODUCTION OF 1-PHTHALIDYL-5-FLUOROURACIL DERIVATIVES AND FORMULATIONS CONTAINING SUCH DERIVATIVES

The present invention provides new phthalidylammonium derivatives which are useful in a new process for producing 1-phthalidyl-5-fluorouracil derivatives, which process comprises reacting the aforesaid phthalidylammonium derivatives with 5-fluorouracil (hereinafter abbreviated to "5-FU"). The 1-phthalidyl-5-fluorouracil compounds produced in the present invention are known to have potent antitumour activity and can be utilized as antitumour agents for humans and animals, and they are particularly useful in the treatment of various malignant tumours. Thus, the 1-phthalidyl-5-fluorouracil derivatives made in the process are particularly useful in the treatment or prophylaxis of metastases of malignant tumours such as, for example, uterine cancer, oesophagus carinoma, cutaneous cancer, gastric cancer, lung cancer, liver cancer, colic and rectum cancer, pancreas cancer, mammary cancer, bladder

cancer, trophoblastic neoplasia, cerebral tumour, lymphosarcoma and leukemia [Japanese Unexamined Patent Publication Nos. 57-16881 and 57-67578].

In the prior art, the 5-fluorouracil derivatives have been prepared in the following ways.

1) Reaction of 5-FU with a 3-halophthalide in the presence of an organic or inorganic base [Japanese Unexamined Patent Publication No. 57-16881].

2) Reaction of 5-FU with a 3-trifluoracetylphthalide [Japanese Patent Application No. 57-181047].

One aspect of the present invention relates to a process for producing a 1-phthalidyl-5-fluorouracil derivative represented by the general formula (I):

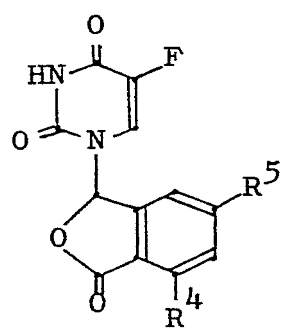

(I)

[wherein $R^4$ and $R^5$ each independently represents hydrogen, trialkylsilyloxy, alkoxy, nitro, cyano, carboxy or alkoxycarbonyl], which comprises reacting a phthalidyl compound represented by the general formula (II):

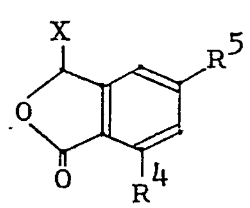

(II)

[wherein X represents a leaving group, such as, for example, halogen, trihalogenoacetyloxy, alkylsulfonyloxy or arylsulfonyloxy] with a tertiary amine represented by the general formula (III):

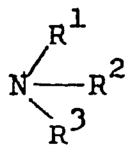

(III)

[wherein $R^1$, $R^2$, and $R^3$ each represents alkyl or $R^1$ represents alkyl and $R^2$ and $R^3$ taken together with the adjacent nitrogen atom form a saturated 5- or 6-membered ring which may contain oxygen or 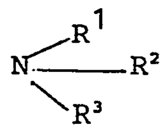 represents quinuclidine]

to yield a phthalidylammonium compound represented by the general formula (IV):

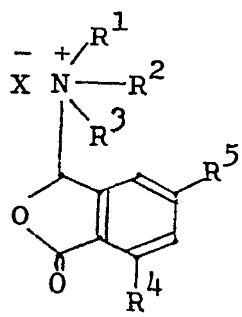

(IV)

[wherein X, $R^1$, $R^2$, $R^3$, $R^4$ and $R^5$ each have the same significance as defined above], and reacting the latter with 5-FU. The present invention also relates to a process for producing a 1-phthalidyl-5-fluorouracil derivative represented by the general formula (I) given above, which comprises adding a tertiary amine represented

by the general formula (III) given above to a phthalidyl compound represented by the general formula (II) given above and then allowing the product to react with 5-FU.

The invention includes the aforementioned phthalidylammonium compounds of formula IV.

In the above general formulae (I) to (IV) the following examples apply:
"halogen" includes fluorine, chlorine, bromine and iodine;
"trihalogenoacetyloxy" incluges e.g. trifluoroacetyloxy, trichloroacetyloxy and tribromoacetyloxy;
"alkylsulfonyloxy" includes $C_1$-$C_5$ alkylsulfonyloxy, e.g. methylsulfonyloxy, ethylsulfonyloxy, propylsulfonyloxy, isopropylsulfonyloxy, butylsulfonyloxy, isobutylsulfonyloxy, t-buytlsulfonyloxy, pentylsulfonyloxy, isopentylsulfonyloxy, neopentylsulfonyloxy or t-pentylsulfonyloxy;
"arylsulfonyloxy" includes e.g. benzenesulfonyloxy, p-toluenesulfonyloxy, p-bromobenzenesulfonyloxy, p-chlorobenzenesulfonyloxy, p-methoxybenzenesulfonyloxy and p-nitrobenzenesulfonyloxy;
"alkyl" includes $C_1$-$C_5$ alkyl, e.g. methyl, ethyl, propyl, isopropyl, butyl, isobutyl, pentyl or isopentyl;
possible saturated 5- and 6-membered rings which are formed together with the adjacent nitrogen atom and which may contain oxygen include e.g. piperidino, pyrrolidino and morpholino;

"trialkylsilyloxy" may be silyloxy substituted by three $C_1$-$C_4$ alkyls which may be the same or different, e.g. trimethylsilyloxy, triethylsilyloxy, tripropylsilyloxy, dimethylsilyloxy, dimethylpropylsilyloxy, diethylmethylsilyloxy, methylethylpropylsilyloxy, dimethylbutylsilyloxy or dimethyl-t-butylsilyloxy;
"alkoxy" may be $C_1$-$C_5$ alkoxy, including e.g. methoxy, ethoxy, propoxy, isopropoxy, butoxy, isobutoxy, t-butoxy, pentyloxy, isopentyloxy, neopentyloxy or t-pentyloxy; and "alkoxycarbonyl" includes such groups having alkoxy groups as mentioned above.

According to the present invention the end objective compounds (I) can easily be produced from starting compounds (II) through the novel intermediates (IV) which are themselves part of the invention.

The intermediates (IV) can be produced by reaction of the starting compounds (II) with tertiary amines (III).

As the tertiary amines (III) which may be used in the reaction, trimethylamine, triethylamine, tri-n-propylamine, tri-n-butylamine, N-methylpiperidine, N-ethylpiperidine, N-methylpyrrolidine, N-ethylpyrrolidine, N-methylmorpholine, N-ethylmorpholine and quinuclidine may be mentioned as examples. The reaction may be conducted in an appropriate solvent with ice-cooling or heating, and usually terminates within a period of from several hours to several days.

As the solvent an aprotic solvent such as, for example, benzene, acetonitrile, tetrahydrofuran, dimethylformamide, dimethylacetamide or dimethylsulfoxide may be used, and in certain cases the reaction may be promoted by the addition of a catalytic amount of an appropriate reagent such as a sodium halide, potassium halide or tetraalkylammonium halide. Compounds of general formula (IV) in which X is halogen can be converted into the corresponding compounds in which X is an alternative group, e.g trihalogenoacetyloxy, alkylsulfonyloxy or arylsulfonyloxy, by reaction with the corresponding silver salt.

The novel intermediates (IV) of the present invention include:

N,N,N-triethyl-3-oxo-1,3-dihydro-1-isobenzofuranyl-ammonium bromide.

N,N,N-triethyl-3-oxo-1,3-dihydro-1-isobenzofuranyl-ammonium chloride,

4-Methyl-4-(3-oxo-1,3-dihydro-1-isobenzofuranyl)-morpholinium bromide,

4-Methyl-4-(3-oxo-1,3-dihydro-1-isobenzofuranyl)-morpholinium chloride, and

4-Ethyl-4-(3-oxo-1,3-dihydro-1-isobenzofuranyl)-morpholinium bromide.

Objective compounds (I) can them be produced by the reaction of the novel intermediates (IV) with 5-FU. The reaction may be conducted in an appropriate solvent in the presence of a base at room temperature or with heating, and usually terminates within a period of from several hours to several days.

As the solvent an aprotic solvent, acetonitrile, tetrahydrofuran, dimethylformamide, dimethylacetamide, dimethylsulfoxide, and the like, may be employed; preferably dimethylformamide or dimethylsulfoxide is used. As the base, an organic base, e.g. triethylamine, tri-n-butylamine, diisopropylethylamine, N-methylpiperidine, N-ethylpiperidine, N-methylpyrrolidine, N-ethylpyrrolidine, N-methylmorpholine, N-ethylmorpholine, quinuclidine, pyridine, dimethylaminopyridine, picoline, chlodidine or dimethylaniline, or an inorganic base, e.g. potassium carbonate or sodium carbonate, may be used.

In the above reaction, the intermediates (IV) which have previously been produced by reaction of the starting compounds (II) with tertiary amines (III) may, without isolation, be allowed to react directly with 5-FU in the presence of the same tertiary amine or an appropriate alternative base. The whole sequence of reactions can be conducted in a single reaction vessel by a simple operation.

The starting compounds (II) are commercially available or can otherwise easily be produced by known methods described in the literature [for example, Japanese Unexamined Patent Publication No. 57-16881].

The process of the present invention is advantageous in its high selectivity for and high yield of the N(1)-substituted-5-fluorouracil accompanied with production of a negligible amount of by-product N(1),N(3)-disubstituted-5-fluorouracil in comparison with the known method described in Japanese Unexamined Patent Publication No. 57-16881 in which 3-halogenophthalides are used as starting compounds and are allowed to react directly with 5-FU, not through intermediates (IV).

The compounds (I) which may be produced by the process of this invention include:

1-(3-Oxo-1,3-dihydro-1-isobenzofuranyl)-5-fluorouracil,

1-(3-Oxo-6-trimethylsilyloxy-1,3-dihydro-1-isobenzofuranyl)-5-fluorouracil,

1-(3-Oxo-6-triethylsilyloxy-1,3-dihydro-1-isobenzofuranyl)-5-fluorouracil,

1-(3-Oxo-6-t-butyldimethylsilyloxy-1,3-dihydro-1-isobenzofuranyl)-5-fluorouracil,

1-(3-Oxo-6-t-butyldiethylsilyloxy-1,3-dihydro-1-isobenzofuranyl)-5-fluorouracil,

1-(3-Oxo-6-methoxy-1,3-dihydro-1-isobenzofuranyl)-5-fluorouracil,

1-(3-Oxo-6-ethoxy-1,3-dihydro-1-isobenzofuranyl)-5-fluorouracil,

1-(3-Oxo-6-nitro-1,3-dihydro-1-isobenzofuranyl)-5-fluorouracil,

1-(3-Oxo-6-cyano-1,3-dihydro-1-isobenzofuranyl)-5-fluorouracil,

1-(3-Oxo-6-carboxy-1,3-dihydro-1-isobenzofuranyl)-5-fluorouracil,

1-(3-Oxo-6-methoxycarbonyl-1,3-dihydro-1-isobenzofuranyl)-5-fluorouracil,

1-(3-Oxo-6-ethoxycarbonyl-1,3-dihydro-1-isobenzofuranyl)-5-fluorouracil,

1-(3-Oxo-4-trimethylsilyloxy-1,3-dihydro-1-isobenzofuranyl)-5-fluorouracil,

1-(3-Oxo-4-t-butyldimethylsilyloxy-1,3-dihydro-1-isobenzofuranyl)-5-fluorouracil,

1-(3-Oxo-4-methoxy-1,3-dihydro-1-isobenzofuranyl)-5-fluorouracil,

1-(3-Oxo-4-ethoxy-1,3-dihydro-1-isobenzofuranyl)-5-fluorouracil,

1-(3-Oxo-4-nitro-1,3-dihydro-1-isobenzofuranyl)-5-fluorouracil,

1-(3-Oxo-4-cyano-1,3-dihydro-1-isobenzofuranyl)-5-fluorouracil,

1-(3-Oxo-4-carboxy-1,3-dihydro-1-isobenzofuranyl)-5-fluorouracil,

1-(3-Oxo-4-methoxycarbonyl-1,3-dihydro-1-isobenzofuranyl)-5-fluorouracil, and

1-(3-Oxo-4-ethoxycarbonyl-1,3-dihydro-1-isobenzofuranyl)-5-fluorouracil.

The invention includes the formulation of compounds (I), which have been produced as above, for pharmaceutical or veterinary use in accordance with the knowledge of the skilled man and his practice. Such formulations may be in unit dosage form and/or include pharmaceutically or veterinarily acceptable diluents, carriers or excipients.

The present invention will now be explained in more detail by the following Examples.

Example 1

(1) Preparation of N,N,N-triethyl-3-oxo-1,3-dihydro-1-isobenzofuranylammonium bromide

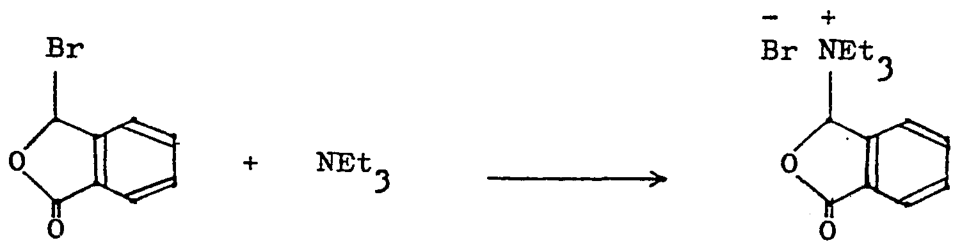

1 2

A mixture of 8.52 g (40 mmol) of 3-bromo-1,3-dihydro-1-isobenzofuranone 1 and 27.87 ml (200 mmol) of triethylamine in 85 ml of tetrahydrofuran is allowed to react at room temperature for 96 hours, and the precipitated crystals are filtered and washed with dichloromethane to give 11.04 g of the title compound 2.

(Yield: 88 %)

m.p. 173 - 174 °C

NMR: δ $d_6$DMSO 1.25 (t, J=8.0Hz, 9H), 3.58 (q, J=8.0Hz, 6H), 7.16 (s, 1H), 7.80-8.23 (m, 4H)

Elemental analysis (%)

Calcd: C, 53.51; H, 6.42; N, 4.46 (for $C_{14}H_{20}NO_2Br$)

Found: C, 53.07; H, 6.42; N, 4.52

(2) Preparation of 1-(3-oxo-1,3-dihydro-1-isobenzofuranyl)-5-fluorouracil

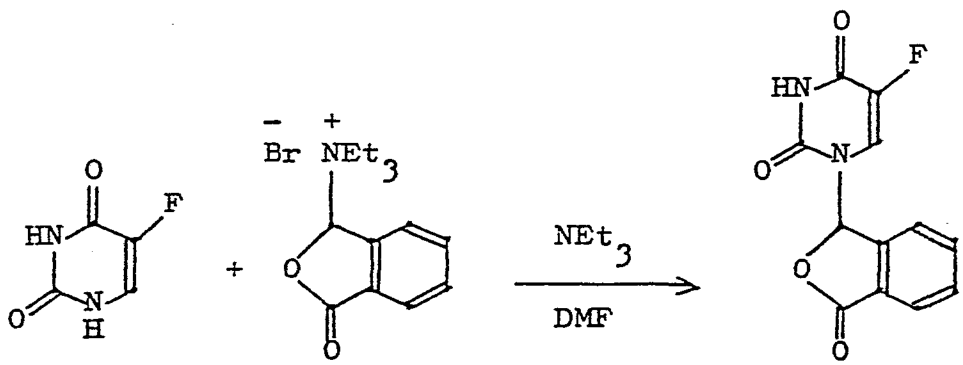


2 3

A solution of 3.14 g (10 mmol) of the compound <u>2</u> provided in the above step(1) and 1.3 g(10 mmol) of 5-FU dissolved in 26 ml of anhydrous dimethylformamide to which is added 0.14 ml (1 mmol) of triethylamine, is allowed to react at room temperature for 15 hours, and the reaction mixture is poured into 260 ml of ice-water containing 5 ml of 2N hydrochloric acid and stirred for 30 minutes. The precipitating crystals are collected by filtration to give 2.44 g of the title compound <u>3</u> (Yield : 93 %). This compound is recrystallized from dimethylsulfoxide-

0112067

water to give white crystals m.p. higher than 290 °C

Elemental analysis (%)

Calcd: C, 54.97; H, 2.69; N, 10.69; F, 7.24 (for $C_{12}H_7O_4N_2F$)

Found: C, 54.95; H, 2.77; N, 10.38; F, 7.64

IR: $\nu_{max}^{KBr}$ 1798, 1715, 1686, 1670 $cm^{-1}$

UV: $\lambda_{max}^{EtOH}$ nm(ε) 227(17,400), 264(10,000)

NMR: δ $d_6$DMSO 4.36 (broad, 1H), 7.5-8.2 (m, 4H), 7.56 (s, 1H), 7.67 (d, J=7Hz, 1H)

Example 2

(1) Preparation of N,N,N-triethyl-3-oxo-1,3-dihydro-1-isobenzofuranylammonium chloride

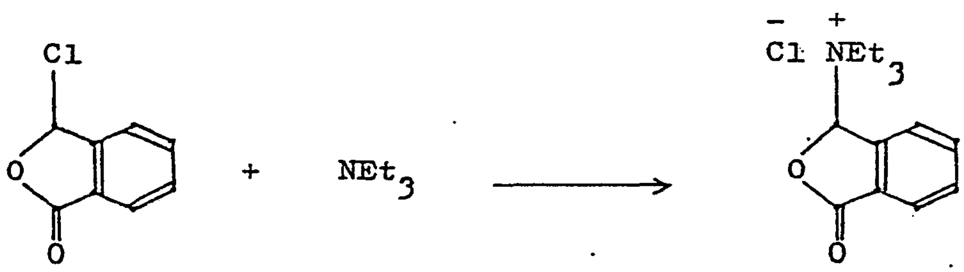

4 5

A mixture of 5.05 g (30 mmol) of 3-chloro-1,3-dihydro-1-isobenzofuranone 4, 12.5 ml (90 mmol) of triethylamine and 225 mg (1.5 mmol) of sodium iodide in 20 ml of acetonitrile is allowed to react with stirring under reflux for 7 hours. The solvent and excess amount of triethylamine are distilled off under reduced pressure,

and the resulting residue is mixed with dichloromethane; the precipitating crystals are collected by filtration to give the title compound 5 as crude crystals.

NMR: δ $d_6$DMSO 1.24 (t, J=8.5Hz, 9H), 3.59 (q, J=8.5Hz, 6H), 7.23 (s, 1H), 7.80-8.27 (m, 4H)

(2) Preparation of 1-(3-oxo-1,3-dihydro-1-isobenzofuranyl)-5-fluorouracil

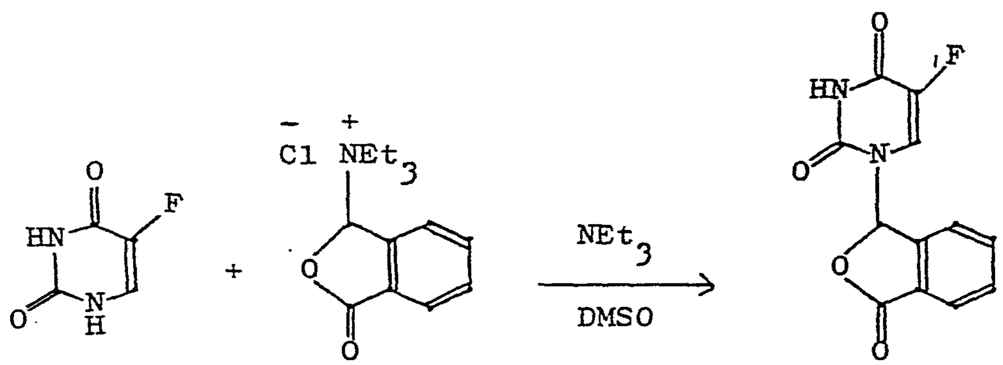


5 3

A solution of 927 mg (7.13 mmol) of 5-FU and 1 equimolar amount of the compound 5 provided in the above step (1) in 6 ml of anhydrous dimethylsulfoxide to which is added 0.5 ml (3.6 mmol) of triethylamine is allowed to react at room temperature for 15 hours, and the reaction mixture is poured into 100 ml of ice-water containing 5 ml of 2N hydrochloric acid, and stirred for 30 minutes. The precipitating crystals are collected by filtration to give 1.76 g of the title compound 3.

(Yield : 94 %).

Example 3

(1) Preparation of 4-methyl-4-(3-oxo-1,3-dihydro-1-isobenzofuranyl)morpholinium bromide

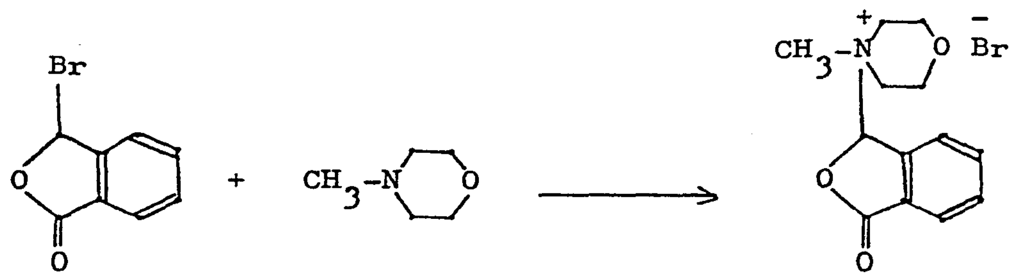

1                                                    6

A mixture of 5.34 g (25 mmol) of 3-bromo-1,3-dihydro-1-isobenzofuranone 1 and 2.75 ml (25 mmol) of N-methylmorpholine in 20 ml of benzene is allowed to react at room temperature for 70 hours, and the precipitating crystals are filtered and washed with benzene to give 6.52 g of the title compound 6 (Yield : 83 %).

m.p. 198-200 °C

Elemental analysis (%)

Calcd: C, 49.69; H, 5.13; N, 4.46; Br, 25.44 (for $C_{13}H_{16}NO_3Br$)

Found: C, 49.14; H, 5.20; N, 4.64; Br, 25.17

NMR: δ $d_6$DMSO 3.03 (s, 3H), 3.4-4.2 (m, 8H), 7.33 (s, 1H), 7.8-8.3 (m, 4H)

(2) Preparation of 1-(3-oxo-1,3-dihydro-1-isobenzofuranyl)-5-fluorouracil

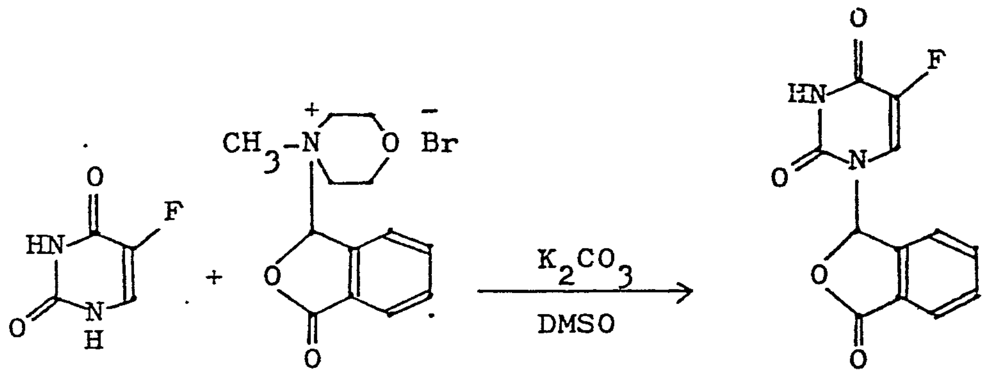


6 3

8.40 g (26.7 mmol) of the compound 6 provided in the above step (1), 3.48 g (26.7 mmol) of 5-FU, and 3.69 g (26.7 mmol) of anhydrous potassium carbonate are added to 35 ml of dimethylsulfoxide, and the mixture is allowed to react stirring well at room temperature for 15 hours. The reaction mixture is poured into 350 ml of ice-water containing 27 ml of 2N hydrochloric acid, and stirred for about 15 minutes; the precipitating crystals are collected by filtration, washed with water (30 ml x 3), methanol (30 ml x 1), and ether (30 ml x 1), and dried to give 6.56 g of the title compound 3 (Yield : 94 %).

Example 4

(1) Preparation of 4-methyl-4-(3-oxo-1,3-dihydro-1-isobenzofuranyl)morpholinium chloride

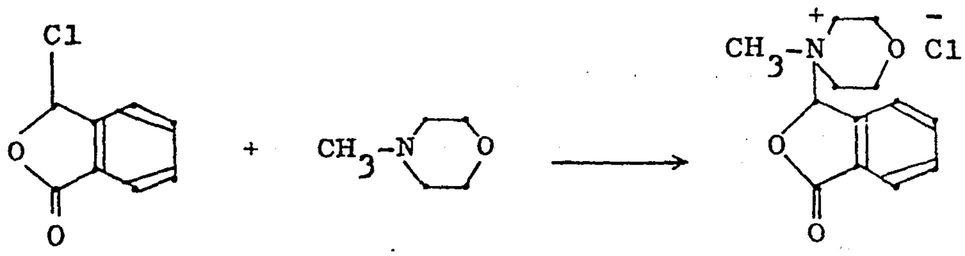

4 7

In the same manner as in Example 3-(1) the title compound 7 is prepared.

NMR: δ $d_6$DMSO 3.03 (s, 3H), 3.7-4.2 (m, 8H), 7.43 (s, 1H), 7.9-8.25 (m, 4H)

(2) Preparation of 1-(3-oxo-1,3-dihydro-1-isobenzofuranyl)-5-fluorouracil

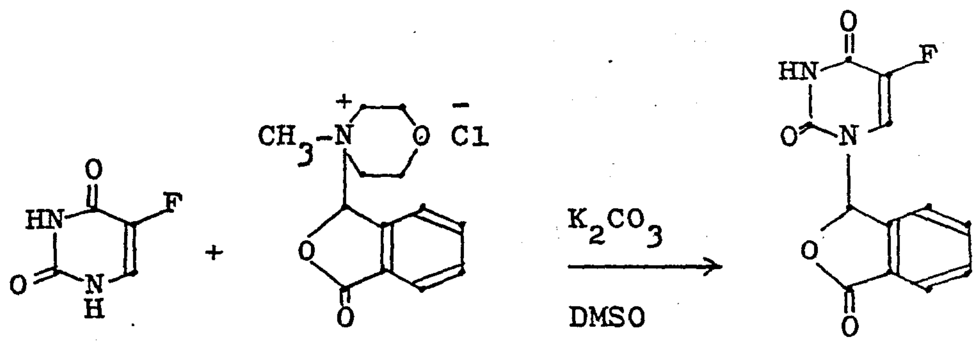


7 3

In the same manner as in Example 3-(2), a mixture of the compound 7 provided in the above step (1) and 5-FU in dimethylsulfoxide is allowed to react in the presence of 1 equimolar amount of anhydrous potassium carbonate at room temperature to give the title compound 3 in good yield.

Example 5

(1) Preparation of N,N,N-triethyl-3-oxo-1,3-dihydro-1-isobenzofuranylammonium trifluoroacetate

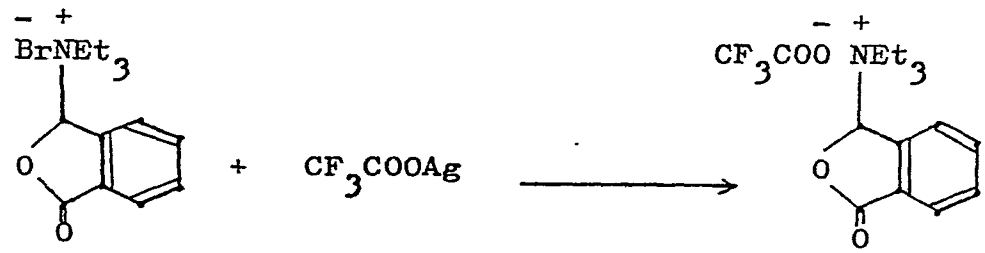


2 8

A mixture of 4.0 g (12.7 mmol) of N,N,N-triethyl-3-oxo-1,3-dihydro-1-isobenzofuranylammonium bromide 2 and 2.81 g (12.7 mmol) of silver trifluoroacetate in acetonitrile is allowed to react at room temperature for 63 hours, and the residue after removal of the solvent under reduced pressure is mixed with dichloromethane. The mixture is filtered in order to remove silver bromide, and the resulting filtrate is concentrated under reduced pressure to give the title compound 8.

NMR: δ $d_6$DMSO 1.23 (t,J=8.0Hz, 9H), 3.56 (q, J=8.0Hz, 6H), 7.07 (s, 1H), 7.80 - 8.27 (m, 4H)

(2) Preparation of 1-(3-oxo-1,3-dihydro-1-isobenzofuranyl)-5-fluorouracil

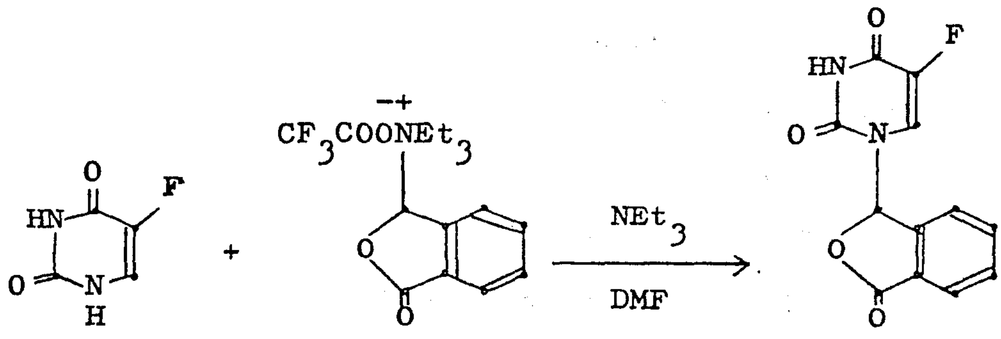


8 3

The compound 8 as crude product provided by the above step (1) and 1.66 g (12.7 mmol) of 5-FU are dissolved in 32 ml of anhydrous dimethylformamide, and the solution to which is added 0.18 ml (1.27 mmol) of triethylamine is allowed to react at room temperature for 15 hours; the reaction mixture is poured into 320 ml of ice-water containing 7 ml of 2N hydrochloric acid and stirred for 30 minutes. The precipitating crystals are filtered to give 3.16 g of the title compound 3 (Yield: 95 %).

Example 6

(1) Preparation of N,N,N-triethyl-3-oxo-1,3-dihydro-1-isobenzofuranylammonium methanesulfonate

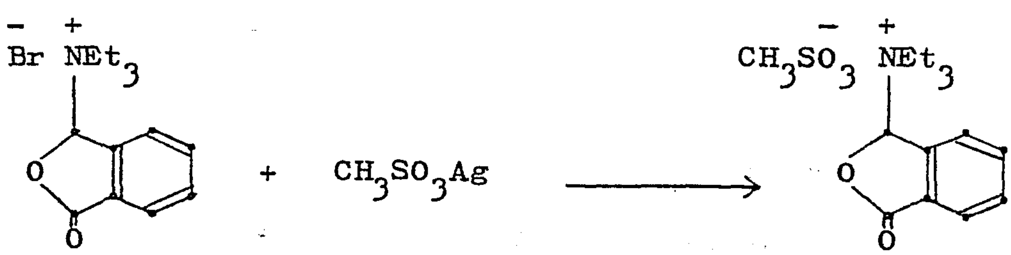

2 9

A mixture of 2.13 g (6.78 mmol) of N,N,N-triethyl-3-oxo-1,3-dihydro-1-isobenzofuranylammonium bromide 2 and 1.38 g (6.78 mmol) of silver methanesulfonate in acetonitrile is allowed to react for 15 hours, and the residue after removal of the solvent under reduced pressure is mixed with dichloromethane and filtered in order to remove silver bromide. The resul ing filtrate is concentrated under reduced pressure to give the title compound 9.

NMR: δ $d_6$DMSO 1.23 (t, J=8.0Hz, 9H), 2.30 (s, 3H), 3.55 (q, J=8.0Hz, 6H), 7.03 (s, 1H), 7.80 - 8.25 (m, 4H)

(2) Preparation of 1-(3-oxo-1,3-dihydro-1-isobenzofuranyl)-5-fluorouracil

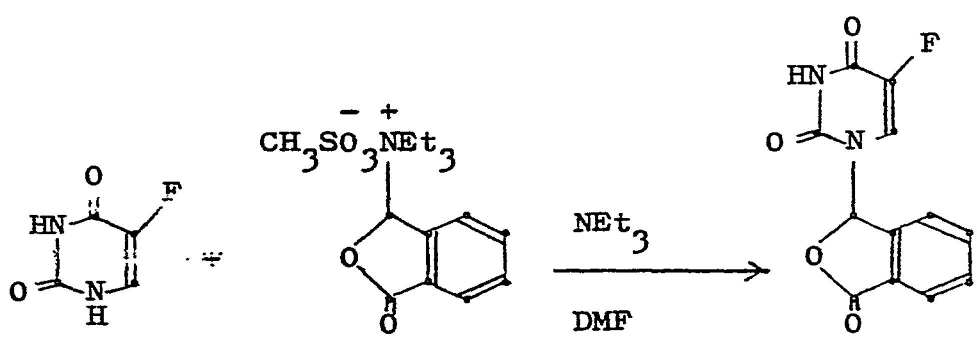


9 3

The compound 9 as crude product provided in the above step (1) and 0.881 g (6.78 mmol) of 5-FU are dissolved in 16 ml of anhydrous dimethylformamide, and the solution to which is added 94 μl (0.678 mmol) of triethylamine is allowed to react at room temperature for 15 hours; the

reaction mixture is poured into 160 ml of ice-water containing 4 ml of 2N hydrochloric acid and stirred for 30 minutes. The precipitating crystals are filtered to give 1.68 g of the title compound 3 (Yield: 95 %).

Example 7

(1) Preparation of N,N,N-triethyl-3-oxo-1,3-dihydro-1-isobenzofuranylammonium p-toluenesulfonate

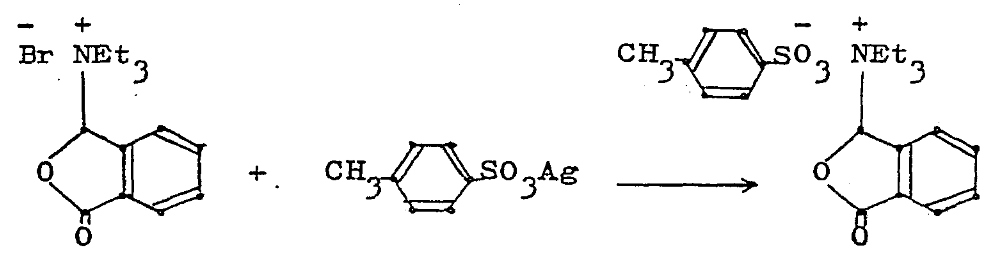

2 10

A mixture of 1.87 g (5.95 mmol) of N,N,N-triethyl-3-oxo-1,3-dihydro-1-isobenzofuranylammonium bromide 2 and 1.62 g (5.95 mmol) of silver p-toluenesulfonate in acetonitrile is allowed to react for 15 hours, and the residue after removal of the solvent under reduced pressure is mixed with dichloromethane and is filtered in order to remove silver bromide. The resulting filtrate is concentrated under reduced pressure to give the title compound 10.

NMR: δ $d_6$DMSO 1.22 (t, J=8.0Hz, 9H), 2.27 (s, 3H), 3.53 (q, J=8.0Hz, 6H), 7.00 (s, 1H), 7.10 (d, J=8.0Hz, 2H), 7.50 (d, J=8.0Hz, 2H), 7.80 - 8.23 (m, 4H)

(2) Preparation of 1-(3-oxo-1,3-dihydro-isobenzofuranyl)-5-fluorouracil

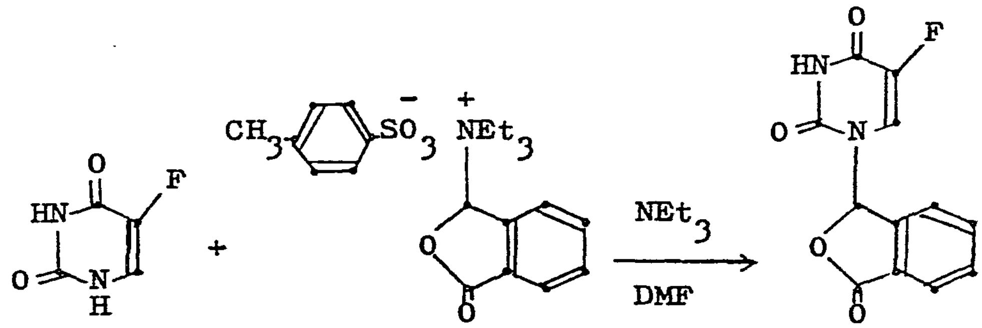


10 3

The compound 10 as crude product provided in the above step (1) and 0.774 g (5.95 mmol) of 5-FU are dissolved in 14 ml of anhydrous dimethylformamide to which is added 83 μl (0.60 mmol) of triethylamine is allowed to react at room temperature for 15 hours; the reaction mixture is poured into 140 ml of ice-water containing 3 ml of 2N hydrochloric acid and stirred for 30 minutes. The precipitating crystals are filtered to give 1.50 g of the title compound 3 (Yield: 96 %).

Example 8

(1) Preparation of 4-ethyl-4-(3-oxo-1,3-dihydro-1-isobenzofuranyl)morpholinium bromide

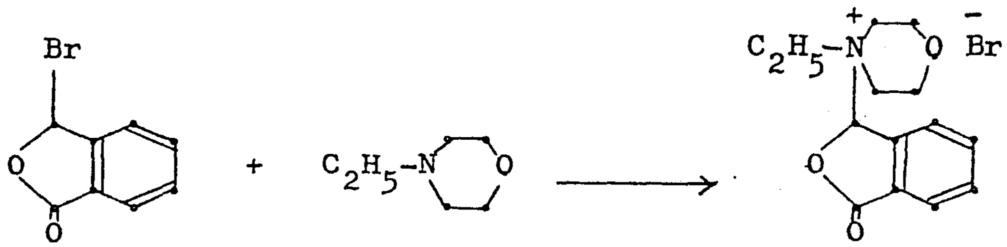

1 11

A solution of 2.13 g (10 mmol) of 3-bromo-1,3-dihydro-1-isobenzofuranone 1 and 1.27 ml (10 mmol) of N-ethylmorpholine in 21 ml of acetonitrile is allowed to react with stirring under reflux for 3 hours. The mixture is cooled and the precipitating crystals are filtered and washed with ether to give 2.36 g of the title compound 11 (Yield: 72 %).

m.p. 163 - 168 °C (dec.)

NMR: δ $d_6$DMSO 1.21 (t, J=7Hz, 3H), 3.5 - 4.2 (m, 10H), 7.30 (s, 1H), 7.83 - 8.30 (m, 4H)

(2) Preparation of 1-(3-oxo-1,3-dihydro-1-isobenzofuranyl)-5-fluorouracil

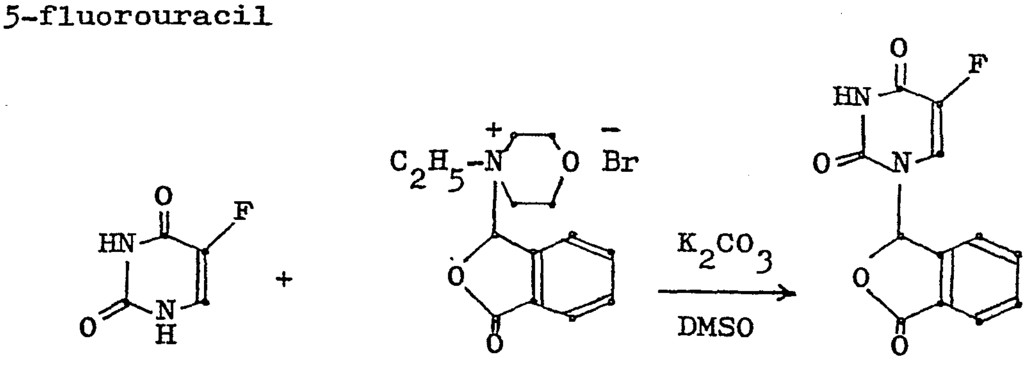


11 3

In the same manner as in Example 3-(2), a mixture of the compound 11 provided by the above step (1) and 5-FU in dimethylsulfoxide is allowed to react in the presence of 1 equimolar amount of anhydrous potassium carbonate at room temperature to give the title compound 3 in good yield.

Example 9

Preparation of 1-(3-oxo-1,3-dihydro-1-isobenzofuranyl)-5-fluorouracil

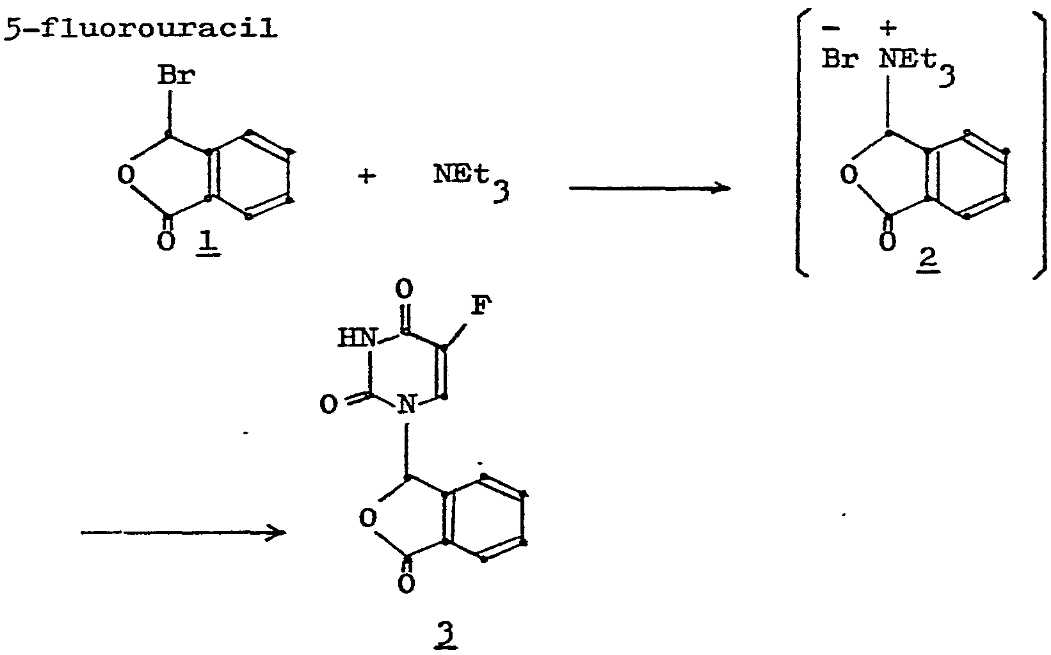

1

2

3

To a solution of 7.67 g (36 mmol) of 3-bromo-1,3-dihydro-1-isobenzofuranone 1 in 60 ml of dimethylformamide is added 5.01 ml (36 mmol) of triethylamine with ice-cooling and the mixture is allowed to react with ice-cooling for 1 hour and then at room temperature for 3 hours. The reaction mixture to which are added 3.90 g (30 mmol) of 5-FU and 1.25 ml (9 mmol) of triethylamine, is allowed

to react with stirring at room temperature for 15 hours. The reaction mixture is poured into ice-water containing 10 ml of 2N hydrochloric acid and stirred for about 15 minutes, and the resulting crystals are collected by filtration, washed with water (50 ml x 3), methanol (50 ml x 1), and ether (50 ml x 1), and dried to give 6.89 g of the title compound 3 (Yield : 88 %).

In the same manner as above, the reaction of compound 2 with 5-FU is carried out in the presence of 1 mole equivalent (to 5-FU) of anhydrous potassium carbonate in place of triethylamine to give a favourable result.

Example 10

Preparation of 1-(3-oxo-1,3-dihydro-1-isobenzofuranyl)-5-fluorouracil

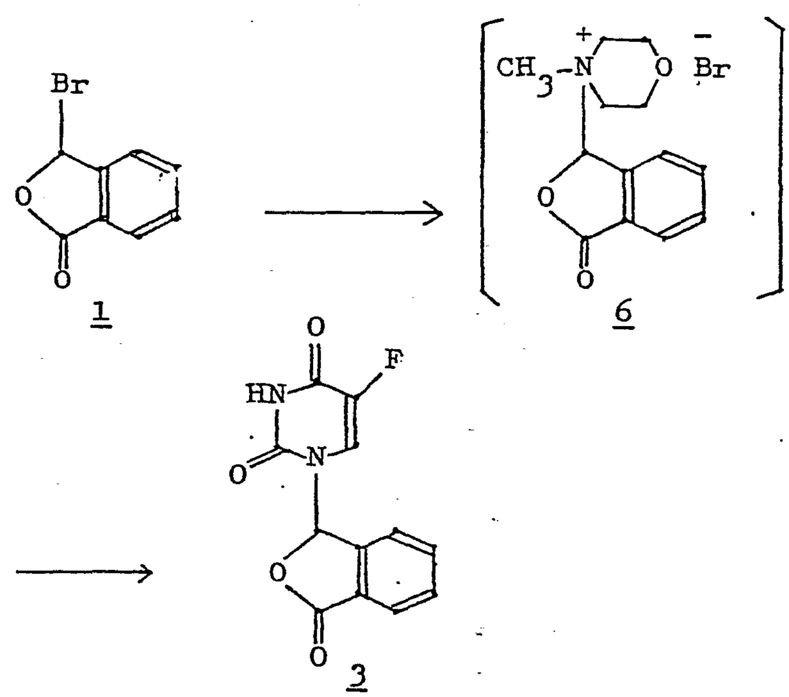

1

6

3

To a solution of 11.5 g (54 mmol) of 3-bromo-1,3-dihydro-1-isobenzofuranone 1 in 100 ml of dimethylformamide is added 5.94 ml (54 mmol) of N-methylmorpholine with ice-cooling, and the mixture is allowed to react with ice-cooling for 15 minutes and then at room temprature for 2 hours with stirring, during which time crystals of the product 4-methyl-4-(3-oxo-1,3-dihydro-1-isobenzofuranyl)morpholinium bromide are precipitated. This suspension is mixed with 5.85 g (45 mmol) of 5-FU and 6.22 g (45 mmol) of anhydrous potassium carbonate, and allowed to react with stirring well for 15 hours. The reaction mixture is poured into about 1 L of ice-water containing 45 ml of 2N hydrochloric acid and stirred for 15 minutes, and the precipitating crystals are collected by filtration, washed with water (50 ml x 3), methanol (50 ml x 1), and ether (50 ml x 1), and dried to give 11.20 g of the title compound 3 (Yield : 95 %).

Example 11

Preparation of 1-(3-oxo-1,3-dihydro-1-isobenzofuranyl)-5-fluorouracil

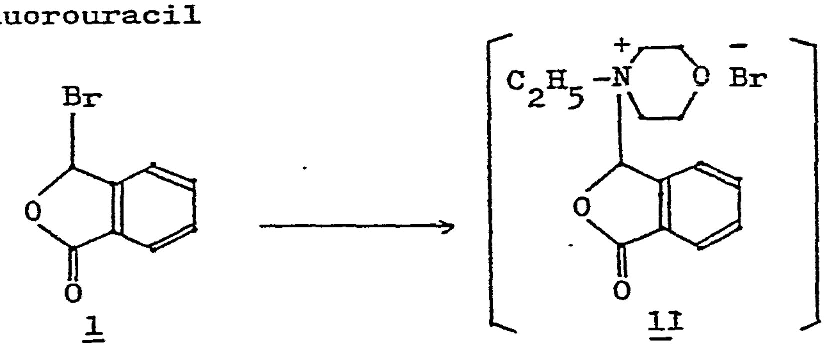

1

11

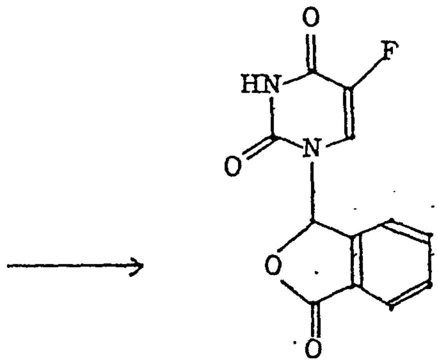

3

To a solution of 6.39 g (30 mmol) of 3-bromo-1,3-dihydro-1-isobenzofuranone 1 in 96 ml of dimethylformamide is added 3.82 ml (30 mmol) of N-ethylmorpholine, and the mixture is allowed to react with stirring for 3 hours. The reaction mixture to which are added 3.9 g (30 mmol) of 5-FU and 0.38 ml (3 mmol) of N-ethylmorpholine is allowed to react with stirring at room temperature for 40 hours. The reaction mixture is poured into 960 ml of ice-water containing 15 ml of 2N hydrochloric acid and stirred for about 15 minutes, and the precipitating crystals are collected by filtration, washed with water (50 ml x 3), methanol (50 ml x 1), and ether (50 ml x 1), and dried to give 5.70 g of the title compound 3 (Yield : 73 %).

In the same manner as above, the reaction of compound 11 with 5-FU is carried out in the presence of 1 mole equivalent (to 5-FU) of anhydrous potassium carbonate in place of N-ethylmorpholine to give a favourable result.

CLAIMS:

1. A compound of the formula:

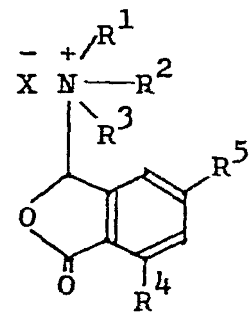

wherein X represents a leaving group, e.g. halogen, trihalogenoacetyloxy, alkylsulfonyloxy or arylsulfonyloxy; $R^1$, $R^2$ and $R^3$ each independently represents alkyl or $R^1$ represents alkyl and $R^2$ and $R^3$ taken together with the adjacent nitrogen atom form a saturated 5- or 6-membered ring which may contain oxygen or 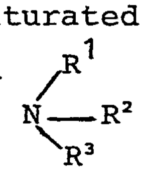 represents quinuclidine; and $R^4$ and $R^5$ each independently represents hydrogen, trialkylsilyloxy, alkoxy, nitro, cyano, carboxy or alkoxycarbonyl.

2. N,N,N-triethyl-3-oxo-1,3-dihydro-1-isobenzofuranylammonium bromide.

3. N,N,N-triethyl-3-oxo-1,3-dihydro-1-isobenzofuranylammonium chloride.

4. 4-methyl-4-(3-oxo-1,3-dihydro-1-isobenzofuranyl)-morpholinium bromide.

5. 4-methyl-4-(3-oxo-1,3-dihydro-1-isobenzofuranyl)-morpholinium chloride.

6. 4-ethyl-4-(3-oxo-1,3-dihydro-1-isobenzofuranyl)-morpholinium bromide.

7. A process for producing a compound as claimed in claim 1 which process comprises reacting a phthalidyl compound of the formula:

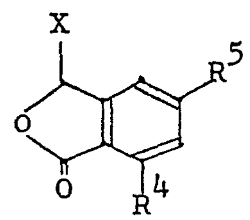

wherein X represents halogen, trihalogenoacetyloxy, alkylsulfonyloxy or arylsulfonyloxy, with a tertiary amine of the formula:

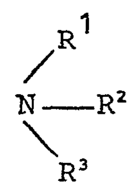

wherein $R^1$, $R^2$ and $R^3$ each independently represents alkyl or $R^1$ represents alkyl and $R^2$ and $R^3$ taken together with the adjacent nitrogen atom form a saturated 5- or 6-membered ring which may contain oxygen or 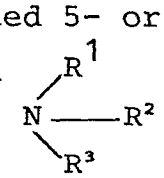 represents quinuclidine; and if X in the resulting phthalidyl ammonium compound is halogen optionally converting it to a compound in which X is other than halogen, e.g. trihalogenoacetyloxy, alkylsulfonyloxy or arylsulfonyloxy by reaction with the corresponding silver salt.

8. A process as claimed in claim 7, wherein the

reaction of the phthalidyl compound with the tertiary amine is carried out in an solvent, e.g. benzene, acetonitrile, tetrahydrofuran, dimethylformamide or dimethylacetamide.

9. A process as claimed in claim 7 or claim 8, wherein the tertiary amine is triethylamine, N-methylmorpholine or N-ethylmorpholine.

10. A process for producing a 1-phthalidyl-5-fluorouracil derivative of the formula:

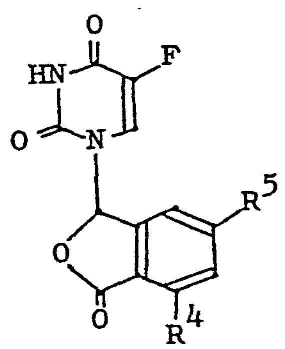

wherein $R^4$ and $R^5$ each independently represents hydrogen, trialkylsilyloxy, alkoxy, nitro, cyano, carboxy or alkoxycarbonyl, which process comprises either (a) reacting a compound as claimed in any one of claims 1 to 6 with 5-fluorouracil; or (b) effecting the steps of a process as defined in any one of claims 7 to 9 and, with or without isolation of a distinct intermediate phthalidyl ammonium compound, reacting the product with 5-fluorouracil.

11. A process as claimed in claim 10, wherein the end product 1-phthalidyl-5-fluorouracil derivative is 1-(3-oxo-1,3-dihydro-1-isobenzofuranyl)-5-fluorouracil.

12. A method of preparing a pharmaceutical or veterinary formulation for use in the treatment or prophylaxis of tumours, which method comprises formulating a 1-phthalidyl-5-fluorouracil derivative as defined in claim 10 and which has been produced by a process as claimed in claim 10 or claim 11 for pharmaceutical or veterinary use, respectively; optionally formulating said derivative with a pharmaceutically or veterinarily acceptable diluent, carrier or excipient and/or rendering said formulation in unit dosage form.